(19) **Europäisches Patentamt**

**European Patent Office**

**Office européen des brevets**

(11) **EP 1 647 910 A1**

(12) **EUROPEAN PATENT APPLICATION**

(43) Date of publication:
**19.04.2006 Bulletin 2006/16**

(51) Int Cl.:
***G06F 19/00*** *(2006.01)*

(21) Application number: **04023115.1**

(22) Date of filing: **29.09.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(71) Applicants:
- **Roche Diagnostics GmbH**
  **68305 Mannheim (DE)**
  Designated Contracting States:
  **DE**
- **F. HOFFMANN-LA ROCHE AG**
  **4070 Basel (CH)**
  Designated Contracting States:
  **AT BE BG CH CY CZ DK EE ES FI FR GB GR HU IE IT LI LU MC NL PL PT RO SE SI SK TR**

(72) Inventor: **Knobel, Rolf**
**6343 Rotkreutz (CH)**

(74) Representative: **Troesch Scheidegger Werner AG**
**Schwäntenmos 14**
**8126 Zumikon (CH)**

Remarks:
Amended claims in accordance with Rule 86 (2) EPC.

(54) **Qualitative analysis of a sample using an algorithm**

(57)    To determine the presence of a specific nucleic acid sequence within a sample, a labelled substance, capable of binding the nucleic acid to be determined, or a labelling substance is added, the substance having the ability to label the nucleic acid or being representative for the nucleic acid. The nucleic acid is amplified, the presence of which is to be determined and the increase of the labelled substance and/or the effect initiated by the labelled substance due to the increase of this specific nucleic acid is determined. The signal increase and/or the effect against time is analysed using a model for determining a deviation from a linear curve.

**EP 1 647 910 A1**

**Description**

The present invention relates to a method for determining the presence of a specific nucleic acid according to the introduction of claim 1, a mathematical model for the detection of a specific nucleic acid within a sample and the use of the mathematical model for the decision whether a specific nucleic acid is present within a sample or not.

Among the number of different analytical methods that detect and quantify nucleic acids based upon the sequences contained in said nucleic acids, polymerase chain reaction (PCR) has become the most powerful and widespread technology, the principles of which are disclosed in the US 4 683 195 and US 4 683 102.

Among a plurality of possible applications of the PCR technique one important field is the detection of DNA sequences being responsible for serious medical defects or diagnosis of serious diseases like Hepatitis, AIDS, Human Papillomavirus (which can cause cervical cancer), Chlamydia trachomatis (which can lead to infertility in women) and the like. PCR technology has become an essential research and diagnostic tool for improving human health and quality of life. PCR technology allows scientist to take a specimen of genetic material, even from just one cell, copy its genetic sequence over and over again and generate a test sample sufficient to detect the presence of absence of specific DNA viruses or bacteria or any particular sequence of genetic materials.

One very important specific field is the testing of blood in blood donation centres where within a short period thousands of samples of blood have to be tested in order to decide whether a specific series of blood may be used or has to be rejected. In particular for blood testing, it is important to have a quick, easy and absolutely reliable test in order to sort out any contaminated blood and to detect a specific DNA sequence which is responsible for a serious disease as e.g. mentioned above.

Therefore, it has been proposed to use labelled substances which can be added to the PCR mixture before amplification of the DNA and to be used to analyse PCR products during amplification. This concept of combining amplification with product analysis has become known as real time PCR which is disclosed e.g. within the WO/97 46707, WO/97 46712 and WO/97 46714. Furthermore, this technique is disclosed within the EP 0 543 942 as well as within the EP 1 041 158 and EP 1 059 523.

Specifically, fluorescent entities are used which are capable of indicating the presence of a specific nucleic acid and which are capable of providing a fluorescent signal related to the amount of specific nucleic acid present within the reaction mixture. In other words, the forming of further nucleic acid chains during the progress of the PCR can be visually followed due to the fluorescent entities.

A specific method in that respect is using so-called TaqMan probes which are short DNA fragments that anneal to a region located between the primer binding sites of the template DNA. The probes bear at different positions a reporter entity and a quencher entity. The polymerases in the PCR solution are able to break down the TaqMan probes during the doubling of the DNA template. In doing so, they free the quencher entity which then migrates away from the influence of the reporter. Hence the fluorescence of the reorter entity is measurable only if the polymerase has in fact copied the desired DNA strand. Each fluorescing molecule of reporter entity represents a DNA strand that has been formed. TaqMan probes can therefore be used to measure and determine the amount of specific DNA formed at any given time.

At present, for determining whether a specific nucleic acid is present within a sample by using so-called TaqMan probes, the change, preferably the increase, of fluorescence is measured and plotted versus time, preferably the number of cycles during the PCR. If the plotted measured points represent more or less a linear base line, the diagnosis usually bears that there is no specific nucleic acid present within the solution. The testing, e.g. of a blood sample, is negative which means that no critical nucleic acid, i.e. DNA or RNA, representing e.g. Hepatitis, AIDS and the like is present. If a deviation of the increase of fluorescence from to the linear base line is observed, which means that the curve does include a so-called elbow deviation, the diagnosis is positive, meaning the tested blood sample is contaminated.

But as the kinetics of PCR reaction is quite complicated, the reaction results require special data analysis because the fluorescence signal level has no simple relation to the amount of input nucleic acid. In the actual used method for diagnosis, in particular of blood samples, some of diagnosis results are judged to be negative which in fact might be positive.

Therefore, one subject of the present invention is to create a method for the detection of a specific nucleic acid in a sample, which method is easy to be executed, completed within a relatively short period, is more reliable and relatively cheap.

Proposed according to the present invention is a method linked to the wording of claim 1.

According to the proposed method, a novel qualitative algorithm is proposed combining the two models of linear versus combined linear and sigmoid curves which are compared statistically. Therefore, by using the PCR technique a labelled substance is added to a sample to be tested containing a sequence complementary to a region of the nucleic acid to be determined to detect whether it is present or not within the mentioned sample. The mixture is maintained under conditions for amplification, e.g. by polymerase chain reaction, and the increase of a signal initiated by the labelled substance and/or the effect initiated by the labelled substance, due to the possible increase of the specific nucleic acid, is measured or determined. The measured increase of signal or effect is plotted against time, e.g. the cycles of the PCR, and the plotted results are analysed by using the mentioned combined regression model.

Compared with the state of the art, the proposed regression model takes into consideration any deflections or deviations of the measured results in relation to the regression model, which means that deflections or deviations of the particular fluorescence signals at each cycle are taken into consideration due to the kinetics of the PCR.

First, a mathematical regression analysis is made with the full data set. A quasi linear regression according to the following formula

$$f(x) = \beta_1 + \beta_2 \cdot x + \beta_3 \cdot s(x)$$

with three regression coefficients is made multiple times. $\beta_1$ is a constant, $\beta_2$ is the linear slope and $\beta_3$ the size of the sigmoid like function s(x) The trial function is a linear curve, combined with a sigmoid curve, with a constant (preset) slope d.

$$s(x) = \frac{1}{(1+\exp(d * (e-x)))}$$

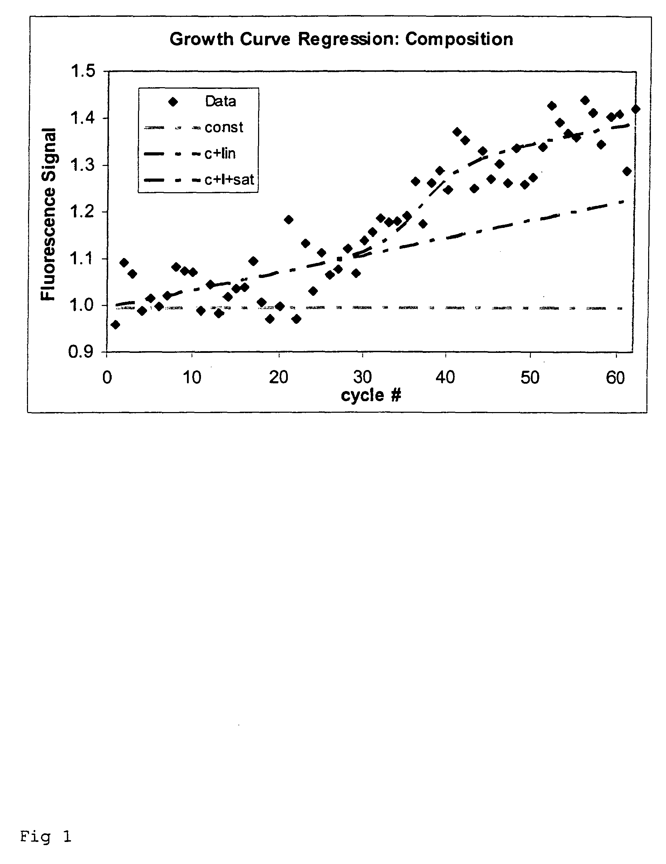

This is made with the inflection points e varying over a preset cycle number range (input parameter). The series of calculated regression coefficients $\beta_3$ are used for further analysis. In the attached Fig. 1 a constant, a linear and a combined sigmoid curve regression are shown representative of the measured fluorescence during a PCR in relation to the cycles.

For the linear and combined curve regression e.g. the following specific mathematical model is proposed:

$$f(x) = \beta_1 + \beta_2 \cdot x + \beta_3 \cdot \frac{1}{1+e^{d \cdot (e-x)}} \cdot$$

If the term $\beta_3 = 0$, then we have a classical linear regression, meaning that we have a straight line. In such a case the diagnosis is quite simple as we have no accelerated increase of the fluorescence and therefore the straight line is representing the basic fluorescence within the mixture. The slope increase may be caused e.g. by changes of the reagents used in amplification, e.g. the "mastermix", changes of the pH-value, changes in temperature of the mixture, etc.

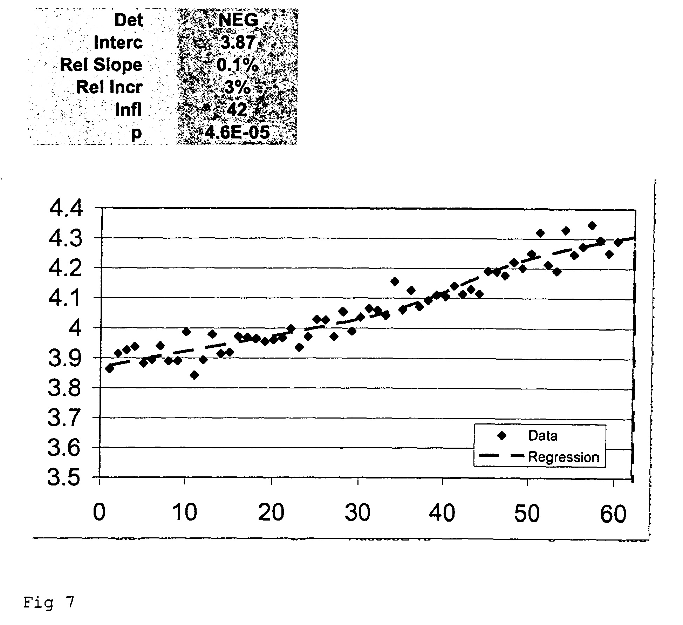

In such a case the diagnosis is simple as the result is negative. The null hypothesis β3= 0 corresponds to no growth present which would be reported as "negative". A positive result is indicated by a fluorescence increase starting at any of the amplification cycles which is above the fluorescence baseline. Taking e.g. Fig. 7 into consideration, it is sometimes very difficult to judge whether a linear or a combined linear and sigmoid curve regression is possible, which means to determine whether β3 is zero or not.

According to the present invention, it is now proposed to further take statistical methods such as e.g. the t-test of the regression coefficient $\beta_3$ into consideration. A statistical hypothesis test is made for the sigmoid coefficient $\beta_3$. The above mentioned null hypothesis is investigated with e.g. a t-test for the ratio between $\beta_3$ and the standard error of $\beta_3$. In this case the t-value t is is a normalized deviation calculated as the quotient of the regression coefficient and its standard error.

$$t = \frac{\beta_3 - 0}{s.e.(\beta_3)}$$

From the inverse Student t-distribution function a statistical false positive (statistical type I error) probability p can be calculated. The statistical type I error p means that the hypothesis is rejected by the methode even it is true in reality. Other common statistical significance criteria (adjusted $R^2$, SIC) lead to similar results. The most significant regression with varying inflection point is chosen based on the smallest t-value of each regression with varying inflection point e for the final result as shown in Fig. 1.

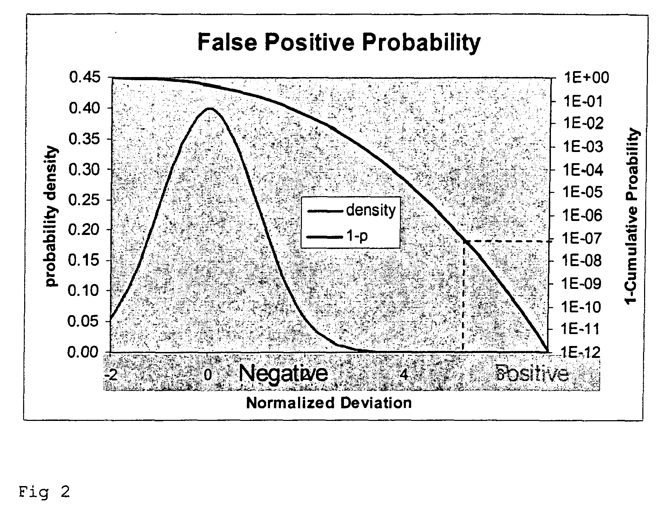

In Fig. 2 a t-test diagram is shown, where line 1 shows the density function of the Student t-distribution. This curve shows the t distribution as function of t in case of negative curves (average(t)=0). The area below that curve represents the probability of a negative showing a special t value or smaller. Line 2 displays the probability p of t being greater than a certain t value (in a logarithmic scale). It is calculated as 100%-cumulative probability of the t distribution function. If e.g. a t-value of 1.5 is determined by the nonlinear regression, then there is a high probability of a correct negative diagnosis and the final result is likely to be negative. But if the value of t is e.g. 6 or 7, then the probability of a wrong positive result is very low. This means that the final result is likely to be positive. The inverse Student t-distribution function relates a t-value of 5 in the graph to a probability p 10^-7 as a possible discrimination value.

To judge now whether the diagnosis is positive or negative, a cut-off value for the statistical false positive probability serves for POSITIVE/NEGATIVE discrimination. With this parameter the sensitivity/specificity of the algorithm can be adjusted.

The borderline between negative and positive is of course an empiric value for each specific application which has to be designated by the execution of a plurality of tests in advance.

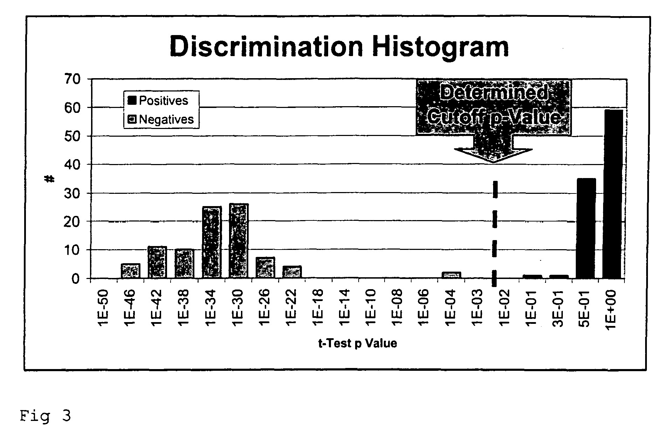

Furthermore, in Fig. 3 there is a discrimination histogram shown being the result of various empirical tests. In other words, if e.g. a t-value of 5 has a p-value of below 1E-03, the result would be positive. Therefore, the probability of a false result is very low. But on the other hand, if a t-value is e.g. 2, having a p-value of e.g. 1E-01, the result would be very likely negative even if the $\beta_3$-value is unequal to 0.

Going back to Fig. 2, these borderlines or determinations of cut-off p-values are indicated with the referential number 5' or 5". Again, the values for these borderlines have to be determined empirically.

The main result of the algorithm is the discrimination between positive and negative. The main result according to the present invention is to judge whether a specific DNA sequence or nucleic acid to be determined within a sample is present or not. In case of the new samples testing, the diagnosis can be done easily, quickly and absolutely safely whether a blood sample is contaminated by the HIV virus or not. Of course, the same diagnosis can be made in relation to other defects such as e.g. the nucleic acids representing Hepatitis B and other diseases as mentioned above. The calculated false positive (type I error) probability itself can also serve to estimate the safety of the result. Additionally, some optional estimations of curve characteristics numbers are extracted from this calculation. They might be used for R&D purposes and possible additional consistency criteria.

Some slight adjustments to the "Sigmoid Regression" algorithm were made to improve the performance. Naturally, negative sigmoid parameters $\beta_3$ are dropped. In case of no positive sigmoid parameter at all, NEGATIVE is reported. Signals more than 20 cycles after inflection are not used for regression to reduce the false detection of negatives with nonlinear drift. Since the algorithm uses all data point, it is robust to spikes. Therefore, no spike detection is required.

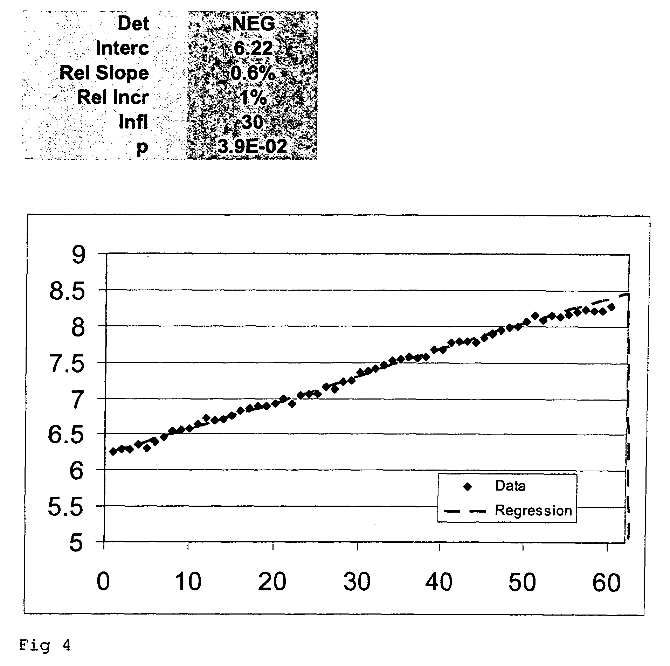

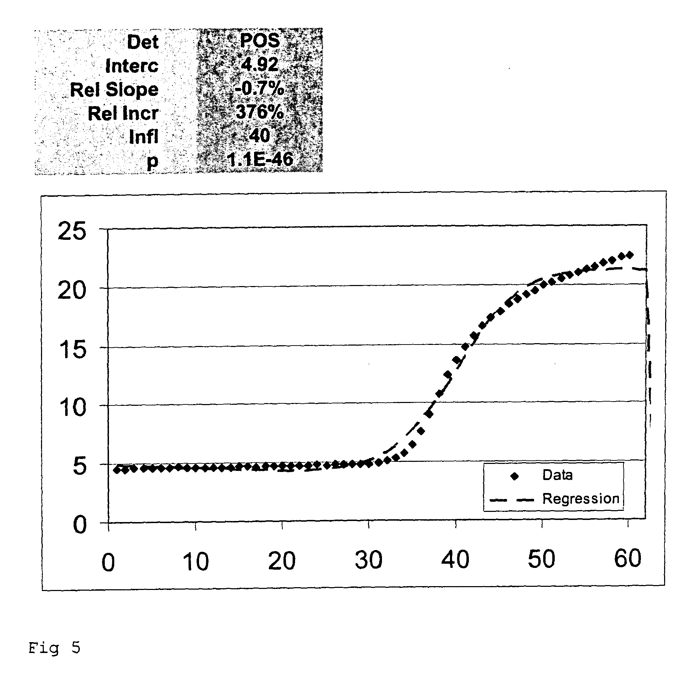

To get a visual impression of the power of the "Sigmoid Regression" algorithm according to the present invention, the attached graphics, shown in Figs. 4 and 5, show two signal curves. In Fig. 4 a clearly negative curve is shown.

In Fig. 5 a reported positive curve is shown.

The $\beta_3$ value of the curve in Fig. 4 is evaluated by the value of the intersection multiplied by the value for the relative increase. In other words, the $\beta_3$ is 6.22 x $10^{-2}$ which is 0.062. As further shown, the p-value is 3.9E-2, meaning the value for a false positive detection would be quite high.

In Fig. 5 the $\beta_3$ is 4.92 x 376% which is equal to 18,49. The probability for a false positive detection is very low, the value is 1.1 E-46. Therefore, a detection of a sample tested and shown according to Fig. 5 is positive and the probability of a wrong diagnosis is rather negligible.

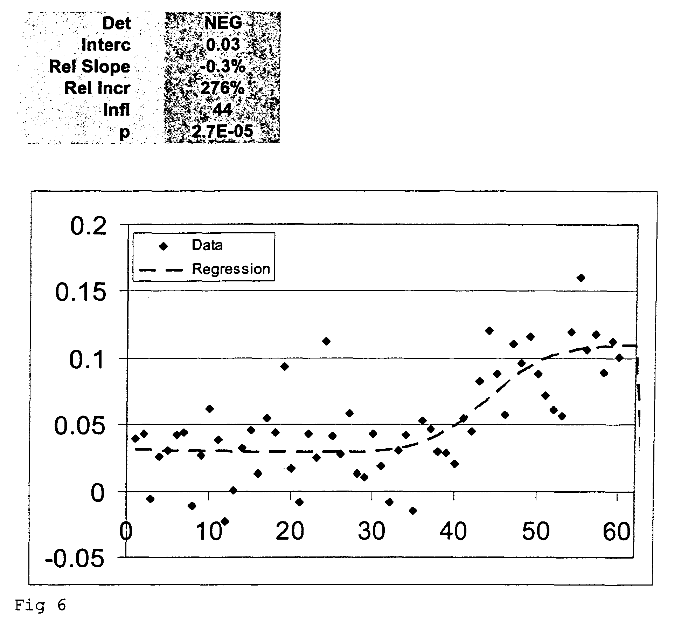

Comparing the two curves shown in Figs. 6 and 7, it is not obvious what results in the curves represent negative results. Investigating the t-test for the two measured parameters $\beta_3$, it can be shown that $\beta_3$-values of the algorithm representing the regression curves of the two Figs. 6 and 7 are within the average deviation which means that the result might be just negative in both cases.

The $\beta_3$ value in Fig. 6 is 0.08 and the p-value is 2.7E-05. Looking at the diagram, these values seem to be rather strange but are explainable due to the tremendous spreading of the measured test points.

In Fig. 7 the $\beta_3$ is 0.12 while the p-value is 4.6E-05.

Even if the t-test value is rather low due to the very high imprecision or the very low $\beta_3$, the diagnosis would be considered as intermittent. Preceding studies lead to the cut-off value to decide on the reported result.

The "Sigmoid Regression" algorithm according to the present invention is the first one developed especially for qualitative detection. Using all data point for calculation, it is statistically well based. However, it is still relatively simple to implement.

Initial algorithm comparison analysis has shown average increased sensitivity from double to 5 fold for low positive samples of five assays. This is reached without affecting specificity.

**Claims**

1. Method for determining the presence of a specific nucleic acid a sample comprising adding a labelled substance capable of binding to the nucleic acid to be determined or a labelling substance, the labelling substance having the ability to label the nucleic acid or being representative for the nucleic acid, amplifying the nucleic acid, the presence of which is to be determined, measuring the change of a signal initiated by the labelled substance or the labelling substance due to the increase of the specific nucleic acid, and analysing the signal change initiated by the labelled substance or the labelling substance against time using a model for determining a deviation from a linear curve.

2. Method according to claim 1 wherein the labelled substance comprises a fluorescent entity.

3. Method according to one of the claims 1 or 2, **characterised in that** a combined regression of a line and a preset curve shape is applied, the non-linear regression term being indicative for the presence of the nucleic acid.

4. Method according to one of the claims 1 or 2, **characterised in that** for the combined regression the following model or approach, respectively, is used:

$$f(x) = \beta_1 + \beta_2 \cdot x + \beta_3 \cdot s(x),$$

where $\beta_1$ and $\beta_2$ are the coefficients for the linear regression and $\beta_3$ is the coefficient for preset nonlinear shape $s(x)$.

5. Method according to claim 4, **characterised in that** a mathematical analysis is made of the nonlinear coefficient $\beta_3$ and the individual deviations of the data from a regression.

6. Method according to claim 5, **characterised in that** as a statistical test like a so-called t-test, a standard average deviation test, a $R^2$ test, a test taking the average quadratic deviation in to consideration or a probability test is used.

7. Method according to claim 6, **characterised in that** a t-test statistical probability p for a type -I- error (of the hypothesis $\beta_3 = 0$) is used as a measure to compare versus a preset cut-off value, wherein lower p values than the cut-off relate to a positive and higher to negative results.

8. Method according to one of the claims 4 to 7, **characterised in that** for the preset curve $s(x)$ the following sigmoid shape is used:

$$s(x) = \frac{1}{1+e^{d \cdot (e-x)}},$$

wherein
d is a preset slope parameter and
e is varying over the measured range and the statistically best regression (minimal type I error p of the t-test) is chosen for result generation.

9. Use of the method according to one of the claims 1 to 8 in a PCR based diagnostic test for detecting pathogens including HIV, Hepatitis B Virus, Hepatitis C Virus, or Human Papillomavirus.

**Amended claims in accordance with Rule 86(2) EPC.**

1. Method for determining the presence of a specific nucleic acid within a sample comprising adding a labelled substance capable of binding to the nucleic acid to be determined or a labelling substance, the labelling substance having the ability to label the nucleic acid or being representative for the nucleic acid, amplifying the nucleic acid, the presence of which is to be determined, measuring the change of a signal initiated by the labelled substance or the labelling substance due to the increase of the specific nucleic acid, and analysing the signal change initiated by the labelled substance or the labelling substance against time using a model for determining a deviation from a linear curve according the following formula:

$$f(x) = \beta_1 + \beta_2 \cdot x + \beta_3 \cdot s(x),$$

where $\beta_1$ and $\beta_2$ are the coefficients for the linear curve and $\beta_3$ is the coefficient for a preset nonlinear shape $s(x)$.

2. Method according to claim 1, **characterised in that** a mathematical analysis is made of the nonlinear coefficient $\beta_3$ and the individual deviations of the data from a regression.

3. Method according to claim 1 or 2, **characterised in that** as a statistical test like a so-called t-test, a standard average deviation test, a R^2 test, a test taking the average quadratic deviation in to consideration or a probability test is used.

4. Method according to one of the claims 1 to 3, **characterised in that** a t-test statistical probability p for a type -I-error (of the hypothesis $\beta_3 = 0$) is used as a measure to compare versus a preset cut-off value, wherein lower p values than the cut-off relate to a positive and higher to negative results.

5. Method according to one of the claims 1 to 4, **characterised in that** for the preset curve $s(x)$ the following sigmoid shape is used.

$$s(x) = \frac{1}{1 + e^{d(e-x)}} \quad \text{`}$$

wherein
d is a preset slope parameter and
e is varying over the measured range and the statistically best regression (minimal type I error p of the t-test) is chosen for result generation.

6. Method according to one of the claims 1 to 5 wherein the labelled substance comprises a fluorescent entity.

7. Use of the method according to one of the claims 1 to 6 in a PCR based diagnostic test for detecting pathogens including HIV, Hepatitis B Virus, Hepatitis C Virus, or Human Papillomavirus.

Fig 1

Fig 2

Fig 3

| Det | NEG |
| --- | --- |
| Interc | 6.22 |
| Rel Slope | 0.6% |
| Rel Incr | 1% |
| Infl | 30 |
| p | 3.9E-02 |

Fig 4

| Det | POS |
| --- | --- |
| Interc | 4.92 |
| Rel Slope | -0.7% |
| Rel Incr | 376% |
| Infl | 40 |
| p | 1.1E-46 |

Fig 5

| Det | NEG |
|---|---|
| Interc | 0.03 |
| Rel Slope | -0.3% |
| Rel Incr | 276% |
| Infl | 44 |
| p | 2.7E-05 |

Fig 6

| Det | NEG |
|---|---|
| Interc | 3.87 |
| Rel Slope | 0.1% |
| Rel Incr | 3% |
| Infl | 42 |
| p | 4.6E-05 |

Fig 7

**European Patent Office**

**EUROPEAN SEARCH REPORT**

Application Number

EP 04 02 3115

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | LIU WEIHONG ET AL: "Validation of a quantitative method for real time PCR kinetics" BIOCHEMICAL AND BIOPHYSICAL RESEARCH COMMUNICATIONS, vol. 294, no. 2, 7 June 2002 (2002-06-07), pages 347-353, XP002319698 ISSN: 0006-291X * the whole document * | 1,2,9 | G06F19/00 |
| X | STUYVER L ET AL: "USING REAL TIME PCR TO DETERMINE ANTI-HIV-1 ACTIVITY AND MITOCHONDRIAL TOXICITY OF NUCLEOSIDE ANALOGS" ANTIVIRAL RESEARCH, ELSEVIER SCIENCE BV., AMSTERDAM, NL, vol. 51, no. 1, July 2001 (2001-07), pages 53-54, XP009017878 ISSN: 0166-3542 | 1,2,9 | |
| Y | * the whole document * | 3-8 | |
| X | YANG JI-HONG ET AL: "Real-time RT-PCR for quantitation of hepatitis C virus RNA" JOURNAL OF VIROLOGICAL METHODS, vol. 102, no. 1-2, April 2002 (2002-04), pages 119-128, XP002319699 ISSN: 0166-0934 * the whole document * | 1,2,9 | TECHNICAL FIELDS SEARCHED (Int.Cl.7) G06F |
| Y | ROYSTON PATRICK: "A strategy for modelling the effect of a continuous covariate in medicine and epidemiology" STATISTICS IN MEDICINE, vol. 19, no. 14, 30 July 2000 (2000-07-30), pages 1831-1847, XP002319700 ISSN: 0277-6715 * the whole document * | 3-8 | |

-/--

The present search report has been drawn up for all claims

| Place of search | Date of completion of the search | Examiner |
|---|---|---|
| The Hague | 2 March 2005 | Gabriels, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 04 02 3115

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | WATERFALL CHRISTY M ET AL: "Kinetic characterisation of primer mismatches in allele-specific PCR: A quantitative assessment." BIOCHEMICAL AND BIOPHYSICAL RESEARCH COMMUNICATIONS, vol. 299, no. 5, 20 December 2002 (2002-12-20), pages 715-722, XP002319701 ISSN: 0006-291X * the whole document * ----- | 3-8 | |
| Y | US 2003/236633 A1 (MEI RUI ET AL) 25 December 2003 (2003-12-25) * the whole document * ----- | 3-8 | |
| | | | TECHNICAL FIELDS SEARCHED (Int.Cl.7) |

The present search report has been drawn up for all claims

| Place of search | Date of completion of the search | Examiner |
|---|---|---|
| The Hague | 2 March 2005 | Gabriels, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 04 02 3115

This annex lists the patent family members relating to the patent documents cited in the above-mentioned European search report.
The members are as contained in the European Patent Office EDP file on
The European Patent Office is in no way liable for these particulars which are merely given for the purpose of information.

02-03-2005

| Patent document cited in search report | Publication date | Patent family member(s) | Publication date |
|---|---|---|---|
| US 2003236633 A1 | 25-12-2003 | US 2003096986 A1<br>US 2003130802 A1<br>AU 4150102 A<br>WO 0242485 A2 | 22-05-2003<br>10-07-2003<br>03-06-2002<br>30-05-2002 |

EPO FORM P0459

For more details about this annex : see Official Journal of the European Patent Office, No. 12/82